# EUROPEAN PATENT SPECIFICATION

(11) **EP 2 338 426 B1**
(45) Date of publication and mention of the grant of the patent: **25.03.2020**
(21) Application number: 09822092.4
(22) Date of filing: 23.10.2009
(51) Int. Cl.: A61B 18/00, A61B 17/32, A61B 90/00, A61B 18/04

(54) **ULTRASONIC TREATMENT DEVICE AND ULTRASONIC OPERATION SYSTEM**
ULTRASCHALLBEHANDLUNGSGERÄT UND ULTRASCHALLBETRIEBSSYSTEM
DISPOSITIF DE TRAITEMENT PAR ULTRASONS, ET SYSTÈME EXPLOITANT LES ULTRASONS

(30) Priority: 23.10.2008 US 256837
(43) Date of publication of application: 29.06.2011
(73) Proprietor: Olympus Corporation, Tokyo 192-8507 (JP)
(72) Inventor: YOSHIMINE, Hideto, Hachioji-shi, Tokyo 192-8507 (JP)
(74) Representative: Schicker, Silvia
(86) International application number: PCT/JP2009/068285
(87) International publication number: WO 2010/047395

(56) References cited:
- EP-A1- 0 968 684
- EP-A1- 1 199 045
- JP-A- 2000 254 136
- JP-A- 2001 258 089
- JP-A- 2006 043 348
- JP-A- 2008 212 693
- JP-T- 2002 514 958
- US-A- 5 387 190
- US-A- 5 669 922

## Description

### Technical Field

The present invention relates to an ultrasonic surgical instrument and an ultrasonic surgical system which are used to perform an operation such as the incision of a living tissue by utilization of ultrasonic vibration.

### Background Art

As one example of a usual ultrasonic surgical instrument which is used to perform an operation such as the incision of a living tissue by utilization of an ultrasonic vibration, for example, an ultrasonic surgical instrument disclosed in Patent Document 1 is generally known. In this ultrasonic surgical instrument, the proximal end of an elongate inserting portion is connected to a treatment portion on a hand side. This treatment portion is provided with an ultrasonic transducer which generates ultrasonic vibration. The distal end of the inserting portion is provided with a treatment portion for treating the living tissue.

The inserting portion has an elongate tubular sheath. Into the sheath, a rod-like vibration transmitting member (a probe) is inserted. The proximal end of the vibration transmitting member is connected to the ultrasonic transducer. Moreover, the ultrasonic vibration generated by the ultrasonic transducer is transmitted to the distal end of the probe. The probe distal end is provided with a hook-like treatment portion.

During the ultrasonic operation, the hook-like portion of the treatment portion at the distal end is hooked on the living tissue, and in a state where tension is imparted to the living tissue, ultrasonic vibration is transmitted to the distal end of the probe. In consequence, the living tissue is incised by the treatment portion of the distal end of the inserting portion, and a bleeding part is coagulated using frictional heat due to contact with the living tissue.

As a further example, Patent Document 2 discloses a surgical blade assembly including a blade having a radially shaped hook that extends from a curved arm and is located at an anti-node of the assembly.

### Prior Art Documents

### Patent Document

[Patent Document 1] Jpn. Pat. Appln. KOKAI Publication No. 2000-254136
[Patent Document 2] U.S. Pat. Appln. Publication No. US 5 669 922 A

### Summary of the Invention

### Technical Problems

When the probe having a hook-shaped distal end, such as the probe disclosed in Patent Document 1, is used for ultrasonic treatment in the state where the hook-shaped treatment portion is engaged with a living tissue, ultrasonic vibration and the load applied at the time of treatment causes a maximal stress at the hook-shaped treatment portion. If the hook-shaped treatment portion has such a shape as to withstand the ultrasonic vibration and the load which may be applied when it is engaged with the living tissue, the distal end of the probe is inevitably large in size, and delicate or fine treatment is hard to perform.

There may be a case where a driving power source incorporates a failure sensing system which detects a failure of an ultrasonic surgical instrument by sensing a change in a vibration system (which ultrasonically vibrates on a driving power) during ultrasonic treatment. However, in the case where the surgical instrument is reduced in size to enable fine treatment with the distal end of the probe, the change which the vibration system undergoes is small even if the distal end of the probe cracks and breaks. Therefore, even if the distal end of the probe breaks and the ultrasonic surgical instrument fails to operate properly, the driving power source of the failure sensing system of the above structure may not be able to sense the failure of the ultrasonic surgical instrument.

The present invention has been conceived in consideration of the above, and is intended to provide an ultrasonic surgical instrument and an ultrasonic surgical system capable of sensing a failure without fail even if an treatment portion is small in size.

### Solution to Problem

An ultrasonic surgical instrument according to one aspect of the present invention comprises the features of claim 1. An ultrasonic surgical instrument may include: a sheath including a distal end and a proximal end; and an ultrasonic probe to be inserted into the sheath, the ultrasonic probe including an interpolating portion positioned in the sheath, and a hook-like treatment portion arranged in a distal end of the interpolating portion and protruding so that the treatment portion protrudes from the sheath, wherein the treatment portion comprises an extending portion which extends obliquely forward and downward from the upper portion of the distal end of the interpolating portion, and has a recess formed under the extending portion.

Preferably, the treatment portion has a distal end hook portion which warps upwards from the distal end of the extending portion.

Preferably, the treatment portion is provided with the extending portion and the distal end hook portion in a balanced state in which the barycentric position of the whole treatment portion substantially matches that of the whole ultrasonic probe.

Preferably, the treatment portion has an axially parallel portion extended substantially in parallel with the axial direction of the ultrasonic probe between the extending portion and the distal end hook portion.

An ultrasonic surgical system may comprise: an ultrasonic transducer configured to generate ultrasonic vibration; an ultrasonic vibration device configured to drive the ultrasonic transducer; a sheath including a distal end and a proximal end; an ultrasonic probe to be inserted into the sheath and the ultrasonic probe including an interpolating portion positioned in the sheath, and a hook-like treatment portion arranged at a distal end of the interpolating portion so that the treatment portion protrudes from the sheath, the treatment portion including an extending portion which extends obliquely forward and downward from the upper portion of the distal end of the interpolating portion, and has a recess formed under the extending portion; a crack sensor configured to sense a crack generated in the ultrasonic probe; and a controller configured to cause the ultrasonic vibration device to stop driving the ultrasonic transducer if the crack sensor senses the crack.

### Advantageous Effects of the Invention

The present invention can provide an ultrasonic surgical instrument capable of sensing a failure without fail even if a small-sized treatment portion breaks.

### Brief Description of Drawings

FIG. 1 is a sectional view showing the overall schematic constitution of an ultrasonic surgical instrument of a first embodiment according to the present invention;
FIG. 2 is a perspective view showing the distal end of a probe unit of the ultrasonic surgical instrument of the first embodiment;
FIG. 3 is a side view showing the distal end of the probe unit of the ultrasonic surgical instrument of the first embodiment;
FIG. 4 is a perspective view showing a distal end treatment portion of a vibration transmitting member of the ultrasonic surgical instrument of the first embodiment;
FIG. 5 is a side view showing a distal end treatment portion of the vibration transmitting member of the ultrasonic surgical instrument of the first embodiment;
FIG. 6 is a characteristic diagram of ultrasonic vibration in a case where a probe distal end of the ultrasonic surgical instrument of the first embodiment is normal;
FIG. 7 is a characteristic diagram of the ultrasonic vibration in a case where the probe distal end of the ultrasonic surgical instrument of the first embodiment is abnormal;
FIG. 8 is a schematic illustration of the entire ultrasonic surgical system provided with the ultrasonic surgical instrument according to the first embodiment; and
FIG. 9 is a block diagram illustrating the schematic structure of the entire ultrasonic surgical system according to the first embodiment.

### Description of Embodiments

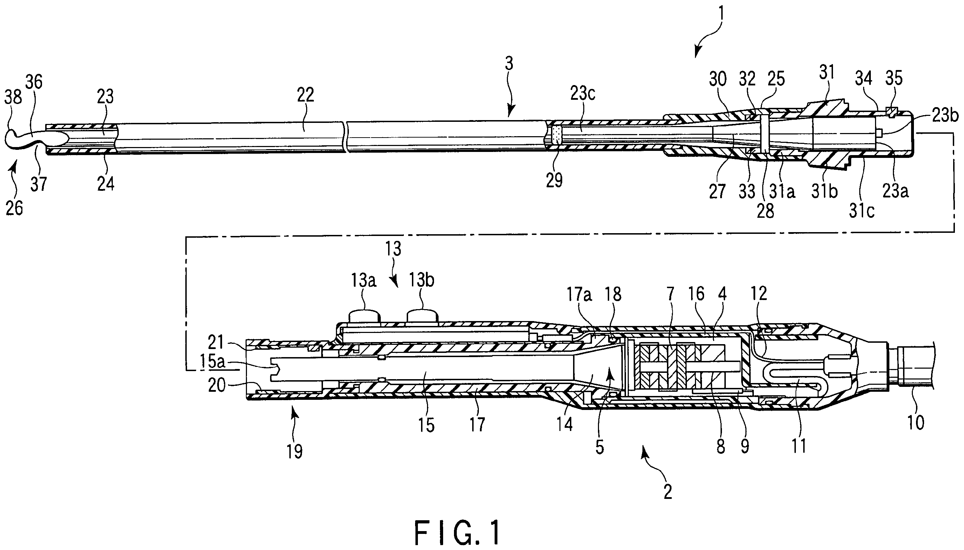

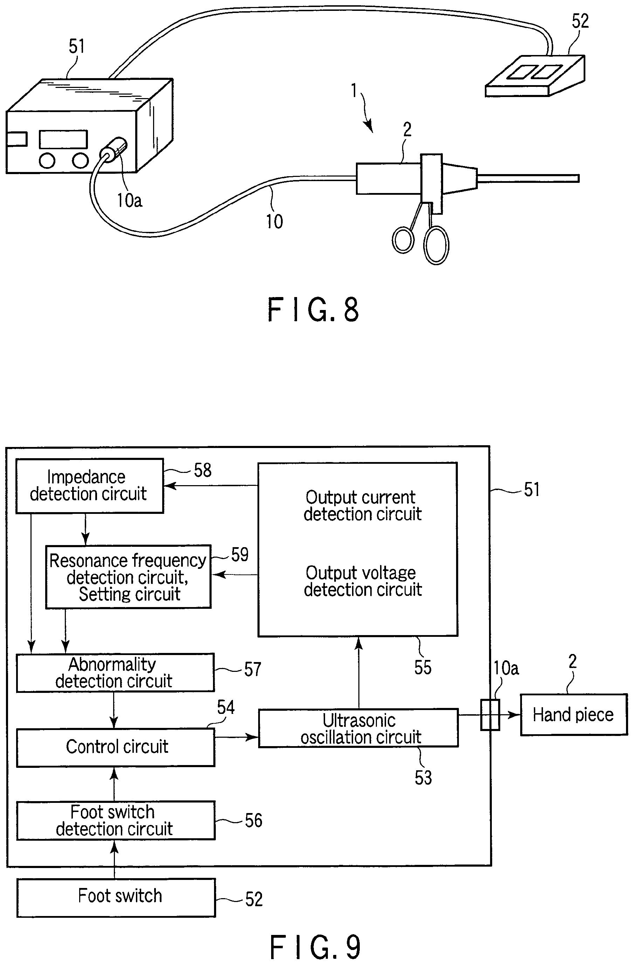

A description will now be given of the first embodiment of the present invention with reference to FIGS. 1 through 9. FIG. 8 is a schematic illustration of the entire ultrasonic surgical system provided with an ultrasonic surgical instrument 1 according to the first embodiment. In FIG. 8, numeral 51 denotes a driving power source. A hand piece 2 of the ultrasonic surgical instrument 1 and a foot switch 52 are connected to the driving power source 51.

FIG. 1 shows the overall schematic constitution of an ultrasonic surgical instrument 1 of the present embodiment. The ultrasonic surgical instrument 1 has a hand piece 2 and a probe unit 3.

The hand piece 2 has a casing 4 and a bolted Langevin type transducer (BLT) 5. The casing 4 is made of an electrically insulating resin material. The casing 4 contains the BLT 5 therein. The BLT 5 has a plurality of piezoelectric elements 7 and two electrodes 8, 9. The two electrodes 8, 9 come in contact with both end faces of the piezoelectric elements 7.

The proximal end of the hand piece 2 is connected to one end of a cord 10. The other end of this cord 10 is electrically connected to the driving power source 51. In the cord 10, an electric wire 11 for the transducer and an electric wire 12 for a switch are arranged. The electric wire 11 for the transducer in the cord 10 is connected to the two electrodes 8, 9, respectively. A driving power is supplied from the driving power source 51 to the BLT 5 via the electric wire 11 for the transducer in the cord 10.

A switch 13 is disposed on the distal end side of the hand piece 2. The switch 13 has, for example, a first switch 13a for driving in a set output state, and a second switch 13b for driving in a maximum output state. The first switch 13a and the second switch 13b are electrically connected to a control circuit in the driving power source 51 via the electric wire 12 for the switch. The first switch 13a makes it possible to drive with a set amplitude, and the second switch 13b makes it possible to drive with a maximum amplitude.

The front portion of the BLT 5 is connected to an output shaft portion 15 via a conical horn 14. The output shaft portion 15 of the BLT 5 is made of a titanium alloy. The distal end of the output shaft portion 15 extends to the vicinity of the distal end of the hand piece 2. The center of the distal end face of the output shaft portion 15 is provided with a recess 15a for connection.

As to the hand piece 2, a transducer cover 16 made of a resin and an output shaft portion cover 17 made of a resin are arranged in the casing 4. The transducer cover 16 is a cover member which covers the plurality of piezoelectric elements 7 of the BLT 5 and the two electrodes 8, 9. The front end of the transducer cover 16 is extended to the position of the horn 14.

The output shaft portion cover 17 is a tubular cover member which covers the output shaft portion 15 of the BLT 5. The proximal end of the output shaft portion cover 17 is provided with a circular connecting portion 17a having a diameter larger than that of another portion. The connecting portion 17a of the output shaft portion cover 17 is extended to a position where the connecting portion is inserted into the transducer cover 16. Moreover, the connecting portion 17a of the output shaft portion cover 17 is connected to the front end of the transducer cover 16 in a state in which the front end of the transducer cover 16 is fitted into the outer peripheral surface of the connecting portion 17a of the output shaft portion cover 17. A seal member such as an O-ring 18 is attached to a bonding face between the connecting portion 17a of the output shaft portion cover 17 and the front end of the transducer cover 16.

The distal end of the hand piece 2 is provided with a probe receiver 19. The probe receiver 19 is a cylindrical member formed of a resin material. The proximal end of the probe receiver 19 is connected in a state in which the proximal end is fitted into the outer peripheral surface of the distal end of the output shaft portion cover 17. The distal end of the probe receiver 19 is extended to a position where the distal end protrudes forwards from the distal end of the output shaft portion 15.

The inner peripheral surface of the probe receiver 19 is provided with a connecting portion 20 detachably connected to the probe unit 3. This connecting portion 20 is formed of, for example, a cam mechanism having a cam groove 21, a screw hole and the like.

The probe unit 3 has a sheath 22, and a vibration transmitting member (an ultrasonic probe) 23 arranged concentrically with this sheath 22. In the sheath 22, the proximal end of a tube 24 formed of an electrically insulating resin material is provided with a cylindrical grip portion 25 to be gripped by a user. The distal end of the grip portion 25 is connected in a state in which the distal end is fitted into the proximal end of the tube 24.

The vibration transmitting member 23 is made of a titanium alloy. The length of the vibration transmitting member 23 is set to an integral multiple of 1/2 of the wavelength of the driving frequency of the BLT 5. The distal end of the vibration transmitting member 23 is provided with a treatment portion 26, described later. The proximal end of the vibration transmitting member 23 is provided with a horn 27 having a conically tapered face. This horn 27 is capable of enlarging the amplitude of the treatment portion 26 to a necessary amplitude. Moreover, the middle portion of the horn 27 of the vibration transmitting member 23 is provided with a flange 28. The flange 28 is arranged in a vibration nodal position. The center of a rear end face 23a of the vibration transmitting member 23 is provided with a protruding portion 23b to be detachably engaged with the connecting recess 15a of the output shaft portion 15.

A plurality of annular lining rubbers 29 are attached to the portion of the vibration transmitting member 23 covered with the tube 24. The lining rubbers 29 are arranged in the vibration nodal positions. The vibration transmitting member 23 and the tube 24 are arranged around the same center via the lining rubbers 29 without being brought into contact with each other.

The grip portion 25 has a front member 30 and a rear member 31. The rear end of the front member 30 is provided with an engagement hole 32 into which the flange 28 is inserted. The inner diameter of this engagement hole 32 is substantially equal to the diameter of the flange 28.

The rear member 31 has three portions; 31a, 31b and 31c having different outer diameters. The three portions 31a, 31b and 31c are the front portion 31a arranged in the axial direction of the sheath 22 on a front side, the middle portion 31b arranged at the middle, and the rear portion 31c arranged on a rear side. The outer diameter of the front portion 31a is set to such a size that the front portion is inserted into the engagement hole 32 of the front member 30. The inner diameter of the front portion 31a is set to a diameter smaller than that of the flange 28. The diameter of the middle portion 31b is set to a diameter larger than the outer diameter of the rear end of the front member 30.

An annular pressing member 33 is arranged in the engagement hole 32 of the front member 30. Moreover, the front end of the flange 28 inserted into the engagement hole 32 of the front member 30 of the grip portion 25 is allowed to abut on the pressing member 33. In this state, the front portion 31a of the rear member 31 is inserted into the engagement hole 32 of the front member 30 to sandwich the flange 28 between the front portion 31a of the rear member 31 and the pressing member 33. In consequence, the sheath 22 and the vibration transmitting member 23 are fixed via the flange 28.

The rear portion 31c on the proximal end of the grip portion 25 is formed into such a size that the rear portion is detachably inserted into the probe receiver 19 of the distal end of the hand piece 2. The rear portion 31c is provided with a connecting portion 34 detachably connected to the probe receiver 19 of the distal end of the hand piece 2. This connecting portion 34 has, for example, an engaging claw 35 to be engaged with the cam groove 21 of the probe receiver 19 of the hand piece 2. When the hand piece 2 is connected to the probe unit 3, the engaging claw 35 of the probe unit 3 is engaged with the cam groove 21 of the probe receiver 19. At this time, the protruding portion 23b of the rear end face 23a of the vibration transmitting member 23 is inserted into the connecting recess 15a of the output shaft portion 15, and detachably engaged with the recess. In this state, the rear end face 23a of the vibration transmitting member 23 and the output end of the output shaft portion 15 of the BLT 5 are pressed on each other, so that the ultrasonic vibration can be transmitted from the BLT 5 to the vibration transmitting member 23. It is to be noted that when the connecting portion 20 of the probe unit 3 is a screw hole, the connecting portion 34 of the grip portion 25 is formed of an external thread to be engaged with the screw hole of the probe unit 3.

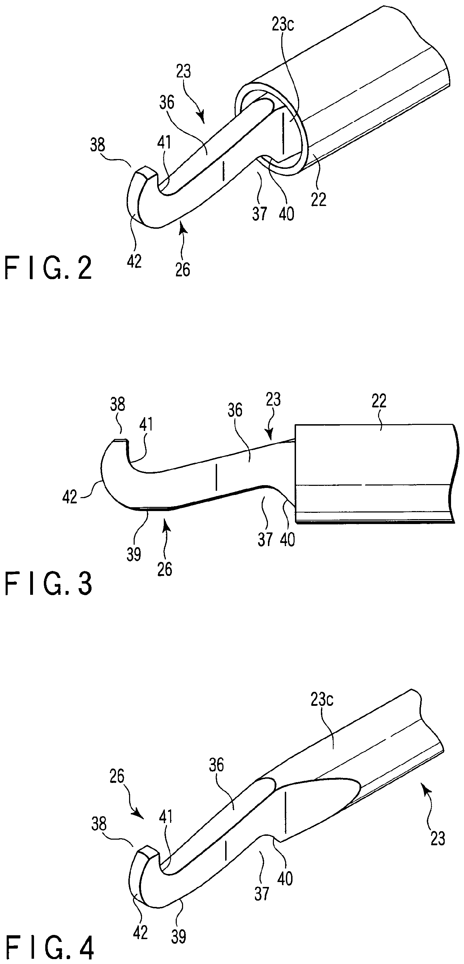

FIGS. 2 and 3 show the distal end of the probe unit 3. The vibration transmitting member 23 has an interpolating portion 23c positioned in the sheath 22, and the hook-like treatment portion 26 arranged on the distal end of the interpolating portion 23c so that the treatment portion protrudes from the sheath 22. The interpolating portion 23c of the vibration transmitting member 23 is formed of a round rod having a substantially circular section. The treatment portion 26 is formed of a plate-like member obtained by processing both side faces of the round rod portion of the interpolating portion 23c so that they are flat and substantially in parallel with each other.

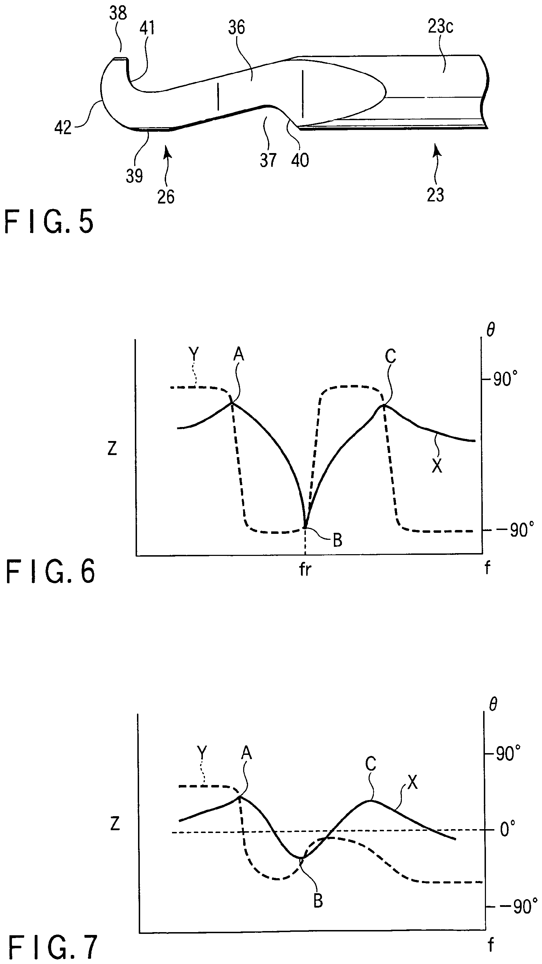

FIGS. 4 and 5 show the treatment portion 26 on the distal end of the vibration transmitting member 23. The treatment portion 26 has an extending portion 36 which extends obliquely forward and downward from the upper portion of the distal end of the interpolating portion 23c. A recess 37 is formed under the extending portion 36.

The treatment portion 26 has a distal end hook portion 38 which warps upwards from the distal end of the extending portion 36. Furthermore, the treatment portion 26 has an axially parallel portion 39 extended substantially in parallel with the axial direction of the vibration transmitting member 23 between the extending portion 36 and the distal end hook portion 38.

Moreover, the proximal end of the extending portion 36 is provided with a smoothly curved face (R-portion) 40 connected to the lower end portion of the distal end of the interpolating portion 23c of the vibration transmitting member 23. This curved face 40 forms the wall face of the recess 37. Furthermore, a portion connecting the axially parallel portion 39 to the distal end hook portion 38 is provided with upper and lower curved portions (an upper curved portion 41, a lower curved portion 42) which are smoothly curved faces.

Furthermore, the distal end hook portion 38 is arranged on the upper surface of the treatment portion 26, and the recess 37 is arranged on the lower surface of the treatment portion. In consequence, the extending portion 36 and the distal end hook portion 38 are formed in a balanced state in which the barycentric position of the whole treatment portion 26 substantially matches that of the whole vibration transmitting member 23.

FIG. 9 is a block diagram illustrating the schematic structure of the entire ultrasonic surgical system according to the first embodiment. A driving power source 51 comprises an ultrasonic oscillation circuit 53 and a control circuit 54. A detection circuit 55, which incorporates an output current detection circuit and an output voltage detection circuit configured to detecting the output current and output voltage of the ultrasonic oscillation circuit 53, is connected to the ultrasonic oscillation circuit 53. A foot switch detection circuit 56, an abnormality detection circuit 57, and the ultrasonic oscillation circuit 53 are connected to the control circuit 54. A foot switch 52 is connected to the foot switch detection circuit 56. An impedance detection circuit 58 and an electric circuit 59, which incorporates a resonance frequency detection circuit and a setting circuit, are connected to the abnormality detection circuit 57. The impedance detection circuit 58 and the electric circuit 59, which incorporates a resonance frequency detection circuit and a setting circuit, are detection circuit 55.

Next, an operation of the present embodiment having the above constitution will be described. To use the ultrasonic surgical instrument 1 of the present embodiment, the ultrasonic surgical instrument 1 is set to a state in which the hand piece 2 is connected to the probe unit 3. At this time, the rear portion 31c of the grip portion 25 of the probe unit 3 is inserted into the probe receiver 19 on the distal end of the hand piece 2. Moreover, the engaging claw 35 of the probe unit 3 is engaged with the cam groove 21 of the probe receiver 19. At this time, the protruding portion 23b of the rear end face 23a of the vibration transmitting member 23 is inserted into the connecting recess 15a of the output shaft portion 15 and detachably engaged with the recess. In this state, the rear end face 23a of the vibration transmitting member 23 and the output end of the output shaft portion 15 of the BLT 5 are pressed on each other, and assembled in a state in which the ultrasonic vibration can be transmitted from the BLT 5 to the vibration transmitting member 23.

To perform an ultrasonic operation, the distal end hook portion 38 of the treatment portion 26 on the distal end of the probe unit 3 is hooked on a living tissue. Moreover, the ultrasonic vibration is transmitted to the treatment portion 26 on the distal end of the probe unit 3 in a state in which tension is imparted to the living tissue. In consequence, the living tissue is incised by the treatment portion 26 on the distal end of the probe unit 3, and a bleeding part is allowed coagulate by frictional heat due to contact with the living tissue.

Furthermore, in the present embodiment, a failure detection system having a constitution in which the change of a vibration system for performing the ultrasonic vibration is detected by the driving power source 51 to detect the failure of the ultrasonic surgical instrument 1 during the ultrasonic operation is incorporated in the driving power source 51.

FIG. 6 is a characteristic diagram of ultrasonic vibration in a case where the treatment portion 26 on the distal end of the probe unit 3 of the ultrasonic surgical instrument 1 is normal. In FIG. 6, the abscissa indicates a frequency (f), the ordinate indicates an impedance (z), a solid-line characteristic curve X indicates the characteristic curve of the ultrasonic vibration, and a dotted-line characteristic curve Y indicates a phase (θ), respectively. Moreover, point B is an impedance resonance point, and points A and C are antiresonance points. Usually, at the resonance point B, the driving power source 51 is controlled into a state in which the treatment portion 26 on the distal end of the probe unit 3 is driven.

Moreover, in the present embodiment, the ultrasonic operation is performed in a state in which the distal end hook portion 38 of the probe unit 3 is hooked on the living tissue. At this time, the treatment portion 26 has the extending portion 36 which extends obliquely forward and downward from the upper portion of the distal end of the interpolating portion 23c. Therefore, when the distal end hook portion 38 of the probe unit 3 is hooked on the living tissue, a maximum stress is generated in the curved face 40 of the proximal end of the extending portion 36 in the treatment portion 26. In this state, when a crack or the like is generated in the treatment portion 26 during an ultrasonic operation, a crack or the like is generated in the curved face 40 of the proximal end of the extending portion 36.

Therefore, in a case where a crack or the like is generated in the treatment portion 26 during an ultrasonic operation, the volume of the distal end of the treatment portion from the portion of the curved face 40 in which the crack is generated increases as compared with a case where the crack is generated in the upper curved portion 41. Consequently, the change in the vibration system (e.g., a resonance frequency (f), an impedance (z), a phase (θ) or the like shown in FIG. 7) in a case where the crack or the like is generated in the treatment portion 26 increases. In this case, the change in the vibration system for performing the ultrasonic vibration is easily detected by the driving power source 51 during the ultrasonic operation, so that the failure of the treatment portion 26 is easily detected during the ultrasonic operation.

If a crack is caused, the hand piece 2 of the ultrasonic surgical instrument 1 can stop outputting signals, for example in the method described below.

When the foot switch 52 is operated by the operator, the foot switch detection circuit 56 receives a detected signal and supplies it to the control circuit 54. In response, the control circuit 54 sets the ultrasonic oscillation circuit 53 in the output state, and the ultrasonic oscillation circuit 53 outputs a current for driving the ultrasonic transducer 5 of the hand piece 2. At the time, the resonance frequency detection circuit 59 detects a phase difference between the output current and the output voltage, based on a signal supplied from the detection circuit 55, in which the output current detection circuit and the output voltage detection circuit are incorporated. The resonance frequency detection circuit 59 regards the frequency at which the phase difference becomes zero as the resonance frequency of the probe (i.e., the vibration transmitting member 23), and sets that frequency as a driving frequency at which the ultrasonic transducer 5 is driven.

If the probe (the vibration transmitting member 23) cracks, the vibration characteristic of the probe (the vibration transmitting member 23) may vary, and a point at which the phase difference between the output current and the output voltage becomes zero may not be present within the driving frequency range of the probe (the vibration transmitting member 23) determined by the ultrasonic oscillation circuit 53. If this happens, the resonance frequency detection circuit of the electric circuit 59 cannot set a resonance frequency. If no resonance frequency can be set, the abnormality detection circuit 57 determines that the probe (vibration transmitting member 23) is in the abnormal state and supplies a control signal to the control circuit 54 to stop the driving of the ultrasonic oscillation circuit 53.

The probe (vibration transmitting member 23) according to the present invention is featured in that a crack which may be caused is limited to the proximal end portion of the treatment portion 26. With this feature, the resonance frequency of the probe (vibration transmitting member 23) varies greatly in response to the generation of a crack, and a point at which the phase difference between the output current and the output voltage becomes zero is present within the driving frequency range of the probe (the vibration transmitting member 23) determined by the ultrasonic oscillation circuit 53. Accordingly, if a crack is generated, the generation of that crack can be sensed very reliably, and the vibration of the ultrasonic transducer 5 inside the hand piece 2 can be stopped.

The present embodiment produces an effect as follows. That is, in the ultrasonic surgical instrument 1 of the present embodiment, the treatment portion 26 is formed so as to include the extending portion 36 which extends obliquely forward and downward from the upper portion of the distal end of the interpolating portion 23c. Consequently, in a case where the distal end hook portion 38 of the probe unit 3 is hooked on the living tissue, the portion of the treatment portion 26 in which the maximum stress is generated can be set to the curved face 40 of the proximal end of the extending portion 36. Therefore, in a case where a crack or the like is generated in the treatment portion 26 during the ultrasonic operation, adjustment can be performed so that a crack or the like is generated in the curved face 40 of the proximal end of the extending portion 36 in which the maximum stress is generated. Consequently, the characteristic change of the vibration system for performing the ultrasonic vibration in a case where a crack or the like is generated in the treatment portion 26 can be increased during the ultrasonic operation, so that any change in the vibration system for performing the ultrasonic vibration can easily be detected by the driving power source 51 during the ultrasonic transducer. Therefore, a state in which a crack or the like is generated in the treatment portion 26 can quickly be detected during an ultrasonic operation, so that even when the size of the treatment portion 26 is decreased for a delicate operation, the driving power source 51 can be stopped before the generation of severe damage such as breakage of the treatment portion 26, to prevent such damage from occurring.

Moreover, in the present embodiment, the treatment portion 26 has the extending portion 36 which extends obliquely forward and downward from the upper portion of the distal end of the interpolating portion 23c, and the recess 37 is formed under the extending portion 36. In consequence, since the recess 37 is provided, the treatment portion 26 can be lightened, so that a moment generated in the treatment portion 26 during the ultrasonic vibration can be decreased. Consequently, a crack or the like is not easily generated in the treatment portion 26 during an ultrasonic operation, which improves the durability.

Furthermore, the distal end hook portion 38 is arranged on the upper surface of the treatment portion 26, and the recess 37 is arranged on the lower surface, so that the treatment portion is formed in a balanced state in which the barycentric position of the whole treatment portion 26 substantially matches that of the whole vibration transmitting member 23. In consequence, there is an effect that lateral vibration transmitted via the vibration transmitting member 23 can be suppressed.

It is to be noted that the present invention is not limited to the above embodiment, and needless to say, the present invention can variously be modified without departing from the scope of the present invention.

### Reference Signs List

22...Sheath, 23c... Interpolating Portion, 26... Treatment Portion, 23...Vibration Transmitting Member (Ultrasonic Probe), 36...Extending Portion, 37...Recess, 38...Distal End Hook Portion.

## Claims

1. An ultrasonic surgical instrument (1) comprising:
an ultrasonic probe (23) which is adapted to transmit ultrasonic vibrations generated in an ultrasonic transducer, and
the ultrasonic probe (23) comprising an interpolating portion (23c) formed of a round rod and a treatment portion (26) which is arranged on the distal end of the interpolating portion (23c),
wherein the treatment portion (26) comprises
an extending portion (36),
a hook portion (38) that warps upward directly from the distal end of the extending portion (36), and
a recess (37) provided on a proximal end side of the treatment portion (26), the recess (37) formed in a portion opposite from a distal end of the hook portion (38) about a longitudinal axis of the ultrasonic probe (23) at a proximal portion of the treatment portion (26), the recess (37) having a curved face (40) forming its wall face, provided in a proximal end of the recess (37), and connected to the recess (37) and a lower portion of the interpolating portion (23c),
**characterised in that**
the extending portion (36) comprises a flatbed upper face portion that extends obliquely downward from the upper portion of the interpolating portion (23c) and a distal end being located at a lowermost portion of the extending portion (36) that extends obliquely, so that when the hook portion (38) is hooked on a living tissue, a maximum stress in the treatment portion (26) is generated in the curved face (40), and
the extending portion (36) is formed with the hook portion (38) in a balanced state in which the barycentric position of the whole treatment portion (26) substantially matches that of the whole ultrasonic probe (23).

## Patentansprüche

1. Chirurgisches Ultraschallinstrument (1), umfassend:
eine Ultraschallsonde (23), die geeignet ist, in einem Ultraschallwandler erzeugte Ultraschallschwingungen zu übertragen, und
wobei die Ultraschallsonde (23) einen Interpolationsabschnitt (23c), der aus einem runden Stab gebildet ist, und einen Behandlungsabschnitt (26) umfasst, der an dem distalen Ende des Interpolationsabschnitts (23c) angeordnet ist,
wobei der Behandlungsabschnitt (26) umfasst
einen sich erstreckenden Abschnitt (36),
einen Hakenabschnitt (38), der sich direkt von dem distalen Ende des sich erstreckenden Abschnitts (36) nach oben windet, und
eine Aussparung (37), die an einer proximalen Endseite des Behandlungsabschnitts (26) vorgesehen ist, wobei die Aussparung (37) in einem Abschnitt gegenüber einem distalen Ende des Hakenabschnitts (38) um eine Längsachse der Ultraschallsonde (23) an einem proximalen Abschnitt des Behandlungsabschnitts (26) ausgebildet ist, wobei die Aussparung eine gekrümmte Fläche (40) aufweist, die ihre Wandfläche bildet, in einem proximalen Ende der Aussparung (37) vorgesehen ist und mit der Aussparung (37) und einem unteren Abschnitt des Interpolationsabschnitts (23c) verbunden ist,
**dadurch gekennzeichnet, dass**
der sich erstreckende Abschnitt (36) einen flachen oberen Flächenabschnitt umfasst, der sich von dem oberen Abschnitt des Interpolationsabschnitts (23c) schräg nach unten erstreckt, und ein distales Ende an einem untersten Abschnitt des sich erstreckenden Abschnitts (36) angeordnet ist, der sich schräg erstreckt, so dass, wenn der Hakenabschnitt (38) an einem lebenden Gewebe eingehakt ist, eine maximale Spannung in dem Behandlungsabschnitt (26) in der gekrümmten Fläche (40) erzeugt wird, und
der sich erstreckende Abschnitt (36) mit dem Hakenabschnitt (38) in einem balancierten Zustand ausgebildet ist, in dem die Schwerpunktsposition des gesamten Behandlungsabschnitts (26) im Wesentlichen mit der der gesamten Ultraschallsonde (23) übereinstimmt.

## Revendications

1. Instrument chirurgical à ultrasons (1) comprenant :
une sonde à ultrasons (23) qui est adaptée pour transmettre des vibrations ultrasonores générées dans un transducteur ultrasonore, et
la sonde à ultrasons (23) comprenant une partie d'interpolation (23c) formée d'une tige ronde et une partie de traitement (26) qui est disposée sur l'extrémité distale de la partie d'interpolation (23c),
dans lequel la partie de traitement (26) comprend
une partie d'extension (36),
une partie crochet (38) qui se déforme vers le haut directement à partir de l'extrémité distale de la partie d'extension (36), et
un évidement (37) ménagé sur un côté d'extrémité proximale de la partie de traitement (26), l'évidement (37) étant formé dans une partie opposée à une extrémité distale de la partie crochet (38) autour d'un axe longitudinal de la sonde à ultrasons (23) au niveau d'une partie proximale de la partie de traitement (26), l'évidement (37) ayant une face incurvée (40) formant sa face de paroi, disposée dans une extrémité proximale de l'évidement (37), et reliée à l'évidement (37) et à une partie inférieure de la partie d'interpolation (23c),
**caractérisé en ce que**
la partie d'extension (36) comprend une partie de face supérieure à plat qui s'étend obliquement vers le bas à partir de la partie supérieure de la partie d'interpolation (23c) et une extrémité distale étant située au niveau de la partie la plus basse de la partie d'extension (36) qui s'étend obliquement, de sorte que lorsque la partie crochet (38) est accrochée à un tissu vivant, une contrainte maximale dans la partie de traitement (26) est générée dans la face incurvée (40), et
la partie d'extension (36) est formée avec la partie crochet (38) dans un état équilibré dans lequel la position barycentrique de l'ensemble de la partie de traitement (26) correspond sensiblement à celle de l'ensemble de la sonde à ultrasons (23).
